# EUROPEAN PATENT APPLICATION

(11) **EP 4 417 194 A1**
(43) Date of publication of application: **21.08.2024**
(21) Application number: 22881423.2
(22) Date of filing: 14.10.2022
(51) Int. Cl.: A61K 9/14

(54) **NANO-MOLECULE AGGREGATE CONSISTING OF ORGANIC MATERIAL, INORGANIC MATERIAL OR SALT THEREOF AND PREPARATION METHOD THEREFOR**

(30) Priority: 14.10.2021 KR 20210136324
(71) Applicant: Scai Therapeutics Co., Ltd., Seoul 06524 (KR)
(72) Inventor: KIM, Chul Hwan, Daejeon 35246 (KR); KIM, Kyoung Hee, Daejeon 35246 (KR)
(74) Representative: dompatent von Kreisler Selting Werner - Partnerschaft von Patent- und Rechtsanwälten mbB
(86) International application number: PCT/KR2022/015656
(87) International publication number: WO 2023/063796

(57) **Abstract**

The present invention relates to a pharmacologically active ingredient-derived molecular association prepared by applying a shear stress to a solution containing a pharmacologically active ingredient.

## Description

### [Technical Field]

The present invention relates to a nano-molecular association consisting of an organic/inorganic material or a salt thereof, and more specifically, to a pharmaceutically active ingredient structure with excellent solubility and permeability to a lipid membrane, which is prepared by applying a shear stress to an organic material, an inorganic material or a salt thereof.

### [Background Art]

In general, in order to effectively deliver pharmacologically active ingredients, so-called active ingredients, in the form of organic/inorganic materials or salts thereof to a target location in the human body, it is known that the following two conditions must be satisfied.

First, the aqueous solubility of the active ingredient must be secured. Since all fluids in the human body are solutions or dispersions based on water, the aqueous solubility or dispersibility must be sufficiently secured, in order for a drug to be delivered to move in the body.

In addition, the permeability of the active ingredient to the hydrophobic membrane must be secured. Since cells in the human body are surrounded by a hydrophobic membrane, such as a phospholipid membrane, the surface properties of drug molecules or drug structures must be hydrophobic to pass through the membrane, or their size must be very small enough to penetrate a cell membrane.

As a conventional method for permeating these drugs through cell membranes in a molecular state, a method of encapsulating a drug in the form of an emulsion or suspension in microspheres using surfactant/polymer structure as a third material has been widely used.

However, the method of encapsulating this drug has limitations in that the encapsulation process is difficult, the encapsulation yield may be low, and the surfactant or polymer component surrounding the drug must be removed or passed through in order for the drug to be released. In addition, since most of the surfaces of cells or tissues that require permeation are composed of phospholipid components and the like, which are lipophilic, there is a problem in that absorption of the drug is not efficient when the drug is hydrophilic.

In order to solve the problems of the encapsulation method of this drug, many researchers have utilized physicochemical bonding by using a third material as mentioned above, rather than utilizing the structure of the molecule itself.

In order to disperse or dissolve drug molecules in water, a method of making the drug molecules polar to induce polar interactions with water molecules, or a method of covalently or ionicly complexing molecules that are very friendly to water, such as PEG, to a part of the molecular structure, or the like may be utilized.

However, these methods had the inconvenience of changing the molecular structure of the drug, there was a problem of price increase due to the preparation process of these molecules, and also, since the changed molecular structure itself was different from the original molecular structure, the initially intended effect of the drug could be displayed only when it was absorbed in the body and then originally returned to its original molecular state. Thus, there was also a limitation in that the drug cannot maintain the original efficacy because the drug molecules do not maintain a plurality of aggregated states without changing their chemical structure. In addition, it was difficult to penetrate the skin gap of 80 nm in size using a drug structure having a size of several hundred nanometers as in the prior art.

Accordingly, various methods for preparing this drug have been studied. In particular, as a method for preparing a drug in nanometer size, there were a top-down method (top-down process) technology such as high pressure homogenization, milling and a piston-gap homogenizer, and bottom-up method (bottom-up process) technology such as precipitation and self-assembly.

The top-down method (top-down process) is a technology for preparing micrometer-sized particles by reducing the size of particles through a method such as milling, and has disadvantages such as increased cost, risk of contamination, and product damage due to repeated milling. On the other hand, the bottom-up method (bottom-up process) is a technology for growing nanometer-sized structures at the atomic or molecular unit, directly opposite to the top-down method, and is a technology proposed as a way to overcome the limitations of the conventional top-down method of reducing the size from a bulk state to a nanometer size. However, at the current technology level, it is difficult to obtain direct results from the economic advantages of technology at the atomic or molecular unit. In addition, it had the advantages of low cost and simple preparation process through crystal growth, which is a conventional method, but had problems in that only crystalline products could be prepared and a separate compound such as a surfactant had to be added in the excessive growth of crystals and their aggregation.

(Patent Document 1) Korean Laid-open Patent Publication No. 10-2011-0053775 (May 24, 2011), A DISPERSION APPARATUS FOR NANO POWDERS USING INTENSITY FOCUSED ULTRASONICS WAVE AND A DISPERSING METHOD USING THEREOF.

### [Disclosure]

### [Technical Problem]

Accordingly, in order to solve the above-mentioned problems, the present inventors have confirmed a phenomenon in which when drug molecules as pharmacologically active ingredients were dissolved in a solvent and then the molecules were brought very close to each other, polar groups in the molecules behaved as if they were a single entity.

In addition, the present inventors have confirmed that when the polar groups interact with each other due to molecular proximity, the drug molecules are hydrophobic as a whole, but the dispersity may be increased because the surface tension decreases as the size of the structure in which these drug molecules are bonded decreases.

From the above, the present inventors have confirmed that small particles at the molecular level may be prepared in the structure of an amorphous, non-crystalline, nano-sized drug, while being prepared in a bottom-up method rather than a top-down method.

In addition, the present inventors have confirmed that a structure in which molecules with new properties are bonded may be prepared through various methods of reducing an intermolecular distance, such as the preparation of molecular structures using pores in powder or the approach of molecules using flexible rolls, and have completed the present invention.

Therefore, it is an object of the present invention to provide a pharmacologically active ingredient structure with increased permeability to a phospholipid membrane, by preparing a structure with a new structure using polar interactions or hydrogen bonds of molecules without changing the chemical structure of pharmacologically active ingredient molecules to make the surface of the structure hydrophobic, and an apparatus capable of preparing the same.

### [Technical Solution]

In order to achieve the above object, the present invention provides a nano-molecular association in which an organic material or an inorganic material is physically bonded, characterized in that: when formed as a composition comprising water in the nano-molecular aggregate, the nano-molecular association in the composition has an aggregated structure; the average particle diameter of the nano-molecular association is 50 nm or less; and when comparing the aqueous solubility A of the pharmacologically active ingredient with the aqueous dispersity B of the nano-molecular aggregate, the value of dispersity B/solubility A is 1.2 or more.

### [Advantageous Effects]

By preparing a molecular association with a new structure using polar interactions or hydrogen bonds without the inconvenience of changing the molecular structure of an organic material, an inorganic material or a salt thereof such as a pharmacologically active ingredient to make the surface of the structure hydrophobic, the present invention may have the advantages of not only increasing the permeability to a phospholipid membrane, but also showing the initially intended drug effect as it is because the original molecule and the structure themselves are the same, and saving the preparation cost because the preparation method is simple.

Due to these advantages, the molecular association of the present invention may be used in various pharmaceutical compositions.

### [Description of Drawings]

Figure 1 shows a schematic diagram of an apparatus for preparing the molecular association according to an embodiment of the present invention.
Figure 2 shows a schematic diagram of an apparatus for preparing the molecular association according to another embodiment of the present invention.
Figure 3 is a graph showing the results of measuring NOESY, which is a two-dimensional nuclear Overhauser effect (NOE) spectrum, for the pharmacologically active ingredients according to an embodiment of the present invention.
Figure 4 is a graph showing the results of measuring NOESY, which is a two-dimensional NOE spectrum, for the molecular association of pharmacologically active ingredients according to an embodiment of the present invention.
Figure 5 is a graph showing XRD measurement results of the molecular aggregates according to another embodiment of the present invention.
Figure 6 is a graph showing DSC measurement results of the molecular aggregates according to another embodiment of the present invention.
Figure 7 shows a TEM picture of the molecular association according to another embodiment of the present invention.
Figure 8 shows 3D hologram pictures of the phospholipid membrane permeation performance of the structures according to another embodiment of the present invention.

### [Best Mode]

Herein is provided a molecular association of an organic material, an inorganic material or a salt thereof, which has the structure of an amorphous, non-crystalline, nano-sized drug, while preparing small particles at the molecular level in a bottom-up method rather than a top-down method, and has increased permeability to a phospholipid membrane, by preparing a molecular association with a new structure using polar interactions or hydrogen bonds without changing the chemical structure of the organic material, inorganic material or salt thereof to make the surface of the molecular association hydrophobic.

Hereinafter, the present invention will be described in more detail.

### Apparatus for preparing a molecular association of an organic material, an inorganic material or a salt thereof

The apparatus for preparing a molecular association of an organic material, an inorganic material or a salt thereof as mentioned in this specification is characterized in that a solution containing an organic material, an inorganic material or a salt thereof is put into the apparatus, and then a shear stress is applied to the solution containing the organic material, inorganic material or salt thereof to prepare a molecular association of the organic material, inorganic material or salt thereof.

As a method for preparing a drug in nanometer size, there were a top-down method technology such as high pressure homogenization, milling and a piston-gap homogenizer, and bottom-up method technology such as precipitation and self-assembly.

The preparation technology of the top-down method is a technology for preparing micrometer-sized particles by reducing the size of particles through a method such as milling, and has disadvantages such as increased cost, risk of contamination, and product damage due to repeated milling.

On the other hand, the preparation technology of the bottom-up method such as precipitation had the advantages of low cost and simple preparation process through crystal growth, but had problems in that only crystalline products could be prepared and a separate compound such as a surfactant had to be added in the excessive growth of crystals and their aggregation.

In addition, in order to well deliver pharmacologically active ingredients in the form of an organic material, an inorganic material or a salt thereof to the target in the human body, the aqueous solubility of the organic material, inorganic material or salt thereof and its permeability to a hydrophobic membrane must be secured. As a conventional method for permeating these drugs through cell membranes in a molecular state, a method of making the drug polar to induce polar interactions with water molecules, or a method of covalently or ionicly complexing molecules that are very friendly to water, such as PEG, to a part of the molecular structure, or the like has been widely used.

However, there were limitations in that these methods had the inconvenience of changing the molecular structure of the drug, and also, since the changed molecular structure itself was different from the original molecular structure, the initially intended effect of the drug could be displayed only when it was absorbed in the body and then originally returned to its original molecular state.

In order to solve the above-mentioned problems, the present inventors have confirmed a phenomenon in which when drug molecules in the form of an organic material, an inorganic material or a salt thereof were dissolved in a solvent and then the molecules were brought very close to each other, polar groups in the molecules interacted to form a molecular association and behaved as if they were a single entity.

From the above, the present inventors have confirmed that when the polar groups interact with each other due to molecular proximity, the drug molecules are hydrophobic as a whole, but the dispersity may be increased because the surface tension decreases as the size of the structure in which these drug molecules are bonded decreases.

The present inventors have confirmed that a molecular association in which molecules with new properties are bonded may be prepared through various methods of reducing an intermolecular distance, such as the preparation of molecular structures using pores in powder or the approach of molecules using flexible rolls, and have completed the present invention.

That is, the present invention is to prepare small particles at the molecular level in the structure of an amorphous, non-crystalline, nano-sized drug, while preparing them in a bottom-up method rather than a top-down method.

First, the present invention provides an apparatus, in which a solution containing an organic material, an inorganic material or a salt thereof is put into the apparatus, and then a shear stress is applied to the solution containing the organic material, inorganic material or salt thereof to prepare a molecular association of the organic material, inorganic material or salt thereof. The apparatus for preparing a molecular association of an organic material, an inorganic material or a salt thereof may be used without particular limitation as long as the molecular association of the organic material, inorganic material or salt thereof according to the present invention may be prepared by applying a shear stress to a solution containing the organic material, inorganic material or salt thereof, but preferably those applying a shear stress using a roll mill process or a ball mill process may be used.

When the solution containing the organic material, inorganic material or salt thereof is prepared, it may be prepared in an oil phase. In this case, as a solvent for preparing it in the oil phase, any one or more of oils derived from grain extracts such as castor oil, MCT oil, soybean oil and peanut oil, and oils derived from extracts of medicinal herbs such as ginseng, camellia, green tea and Korean angelica root, showing pharmacological effects, may be used, but are not necessarily limited thereto.

In addition, in the present invention, the organic material, inorganic material or salt thereof may be used as a pharmacologically active ingredient, and may be used without particular limitation as long as it is a pharmaceutically useful material or a material having a medical effect. As an example, cyclosporin A, paclitaxel, docetaxel, decursin, meloxicam, itraconazole, celecoxib, capecitabine, travo, prost, isoflavone, diclofenac sodium, tyrosine kinase inhibitors such as sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, various chokic acids such as deoxycholic acid and ursodeoxycholic acid, or sodium or potassium salts thereof; steroids such as predimesolone substituted with fluorine or present as hydrogen; fragrance oils such as eucalyptus oil, lavender oil, lemon oil, sandalwood oil, rosemary oil, chamomile oil, cinnamon oil and orange oil; alpha-bisabolol, vitamin A (retinol), vitamin E, tocopheryl acetate, vitamin D, vitamin F or derivatives thereof, or the like may be used, or a combination thereof may be used.

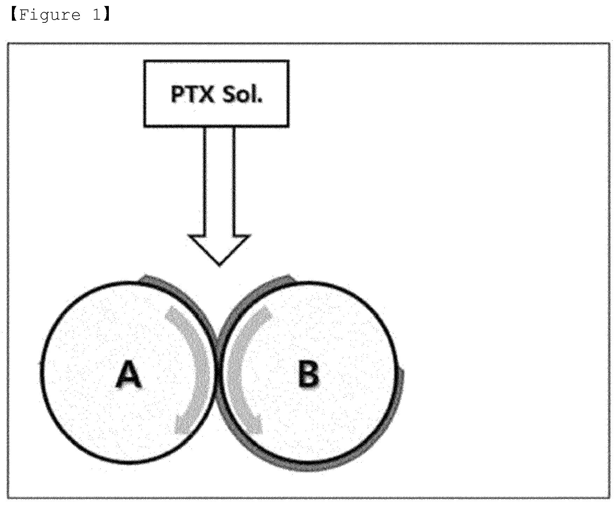

As a specific embodiment of the apparatus for applying a shear stress, a schematic diagram for explaining an apparatus for preparing the molecular association of an organic material, an inorganic material or a salt thereof according to an embodiment of the present invention is shown in Figure 1.

As shown in Figure 1, the apparatus for preparing the molecular association of an an organic material, an inorganic material or a salt thereof according to an embodiment of the present invention may be provided with a plurality of rolls so as to apply a shear stress to the solution containing the organic material, inorganic material or salt thereof. In this case, the plurality of rolls may be two rolls facing each other, and may further include one or several rolls therein.

Specifically, the solution containing the organic material, inorganic material or salt thereof (for example, indicated as PTX Sol. in Figure 1) may be put between two rolls facing each other (indicated as roll A and roll B in Figure 1) in the apparatus. As the two rolls facing each other rotate with respect to the solution containing the organic material, inorganic material or salt thereof put between the two rolls, a shear stress is applied to the organic material, inorganic material or salt thereof contained in the solution containing the organic material, inorganic material or salt thereof, so that the molecular association of the organic material, inorganic material or salt thereof according to the present invention may be prepared.

In the apparatus for preparing the molecular association of an organic material, an inorganic material or a salt thereof according to an embodiment of the present invention, the distance between the two rolls facing each other may be set to 0.5 to 1000 um. The distance between the two rolls facing each other, for example, may be set to 0.5 um or more, 1 um or more, 2 um or more, 3 um or more, 4 um or more, 5 um or more, or 10 um or more, and may be set to 1000 um or less, 900 um or less, 800 um or less, 700 um or less, 600 um or less, 500 um or less, 400 µm or less, 300 µm or less, 200 µm or less, or 100 µm or less, and preferably may be set to 10 to 100 µm. If the distance between the two rolls facing each other is less than 0.5 µm, the discharge amount between the rolls is too small, resulting in a problem of production speed, and if it is more than 1000 µm, the shear stress or compressive stress is too small, resulting in a problem that formation of particles is not easy.

In the case of passing the solution between the rolls as described above, excessive force is applied between the molecules, so that the milling of the top-down method does not occur, but the aggregated particle structure between each molecule is created in the bottom-up method.

In addition, the apparatus for preparing the molecular association according to an embodiment of the present invention may rotate the two rolls facing each other at different speeds, in order to more efficiently transfer a shear stress to the organic material, inorganic material or salt thereof contained in the solution. In this case, the rotational speed of any one of the two rolls facing each other may be 50 to 250 rpm, and the rotational speed of the other may be 200 to 500 rpm. Alternatively, the rotational speed of each of the two rolls facing each other may rotate at a ratio of 1:1.5 to 1:5.

In addition, in the apparatus for preparing the molecular association according to an embodiment of the present invention, the rotational direction of the two rolls facing each other may be set to a co-current direction having the same rotational direction, or may be set to a counter-current direction having different rotational directions.

In addition, the apparatus for preparing the molecular association according to an embodiment of the present invention may repeatedly apply a shear stress or compressive stress to the contents discharged once several times.

In addition, the present invention provides an apparatus, in which a solution containing an organic material, an inorganic material or a salt thereof is put into the apparatus in a first side direction, and another solution is put in a second side direction facing the first side direction, and then a shear stress is applied to prepare a molecular aggregate. The apparatus for preparing the molecular association of the present invention may also be used without particular limitation as long as the molecular association of the present invention may be prepared by applying a shear stress to the solution containing the organic material, inorganic material or salt thereof, but preferably those applying a shear stress using a roll mill process or a ball mill process may be used.

In the present invention, the pharmacologically active ingredients may be the same as mentioned above.

In the present invention, as the water-soluble compounds, any one or more selected from the group consisting of citric acid, carbonic acid, lactic acid, acetic acid, phosphoric acid, ascorbic acid, malic acid, tartaric acid, glutaric acid, succinic acid, maleic acid, fumaric acid, malonic acid, HCl, H₂SO₄, NaH₂PO₄, NaHCO₃, KHCO₃, Na₂CO₃, K₂CO₃, Na₃PO₄, K₃PO₄, NaH₂PO₄, NH₄OH, sodium acetate (NaOAc), KOH, NaOH and Ca(OH)₂ may be used.

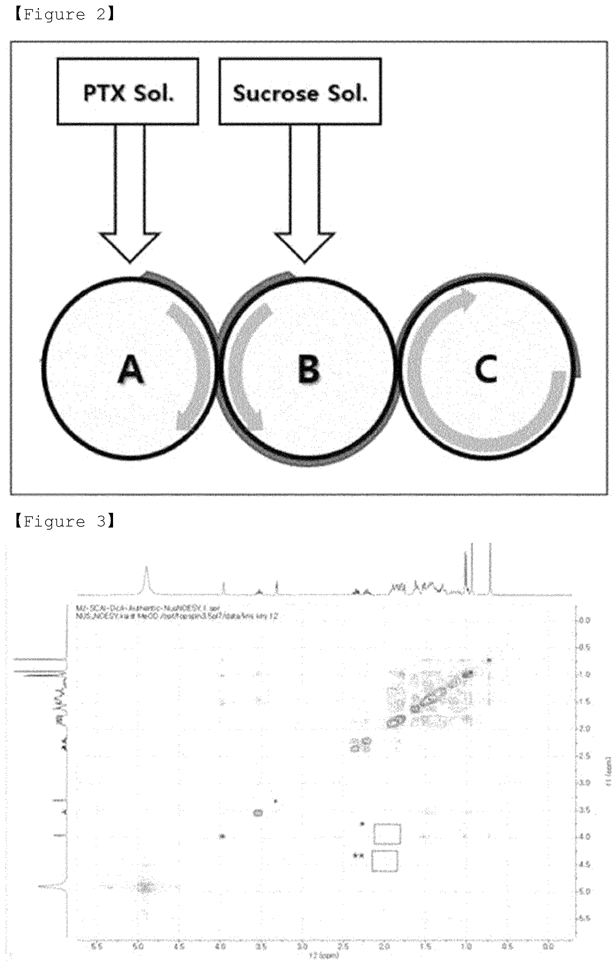

As a specific embodiment of the apparatus for applying a shear stress, a schematic diagram for explaining an apparatus for preparing the molecular association according to an embodiment of the present invention is shown in Figure 2.

As shown in Figure 2, the apparatus for preparing the molecular association according to an embodiment of the present invention may be provided with a plurality of rolls so as to apply a shear stress to a solution containing an organic material, an inorganic material or a salt thereof. In this case, the plurality of rolls may be two rolls facing each other (roll A, roll B), and may further include one or several rolls (roll C) as shown in Figure 2.

Specifically, the solution containing the organic material, inorganic material or salt thereof (for example, indicated as PTX Sol. in Figure 2) may be put into the first roll side, and the solution containing the water-soluble compounds (for example, indicated as Sucrose Sol. in Figure 2) may be put into the side of the second roll facing the first roll.

As the two rolls facing each other rotate with respect to the solution containing the organic material, inorganic material or salt thereof and the solution containing the water-soluble compounds, respectively put in different directions of the two rolls, a shear stress is applied to the organic material, inorganic material or salt thereof and the water-soluble compounds contained in these solutions, so that the molecular association of the organic material, inorganic material or salt thereof according to the present invention may be prepared.

In the apparatus for preparing the molecular association according to an embodiment of the present invention, the distance between the two rolls facing each other may be set to 0.5 to 1000 µm. The distance between the two rolls facing each other, for example, may be set to 0.5 µm or more, 1 µm or more, 2 µm or more, 3 µm or more, 4 µm or more, or 5 µm or more, and may be set to 1000 µm or less, 900 µm or less, 800 µm or less, 700 µm or less, 600 µm or less, or 500 µm or less, and preferably may be set to 5 to 500 µm.

If the distance between the two rolls facing each other is less than 0.5 um, the discharge amount between the rolls is too small, resulting in a problem of production speed, and if it is more than 1000 µm, the shear stress or compressive stress is too small, resulting in a problem that formation of particles is not easy.

In addition, the two rolls facing each other may rotate at different speeds, in order to more efficiently transfer a shear stress to the organic material, inorganic material or salt thereof contained in the solution. In this case, the rotational speed of any one of the two rolls facing each other may be 50 to 150 rpm, and the rotational speed of the other may be 200 to 500 rpm. Alternatively, the rotational speed of each of the two rolls facing each other may rotate at a ratio of 1:1.5 to 1:5.

In addition, in the apparatus for preparing the molecular association according to an embodiment of the present invention, the rotational direction of the two rolls facing each other may be set to a co-current direction having the same rotational direction, or may be set to a counter-current direction having different rotational directions.

In addition, the apparatus for preparing the molecular association according to an embodiment of the present invention may repeatedly apply a shear stress or compressive stress to the contents discharged once several times.

In the present invention, the apparatus for preparing the molecular association may further be provided with a third roll for re-applying a shear stress to the solutions passing between the first roll and the second roll. A shear stress may be applied between the second roll and the third roll, and the distance between the second roll and the third roll facing each other may be set to 0.5 to 1000 µm. The distance between the two rolls facing each other, for example, may be set to 0.5 µm or more, 1 µm or more, 2 µm or more, 3 µm or more, 4 µm or more, or 5 µm or more, and may be set to 1000 µm or less, 900 µm or less, 800 µm or less, 700 µm or less, 600 µm or less, or 500 µm or less, and preferably may be set to 5 to 500 µm. If the distance between the second roll and the third roll is less than 0.5 µm, the discharge amount between the rolls is too small, resulting in a problem of production speed, and if it is more than 1000 µm, the shear stress or compressive stress is too small, resulting in a problem that formation of particles is not easy.

In addition, the second roll and the third roll may rotate at different speeds, in order to more efficiently transfer a shear stress. In this case, the rotational speed of any one of the second roll and the third roll may be 200 to 500 rpm, and the rotational speed of the other may be 600 to 1200 rpm. Alternatively, the rotational speed of each of the second roll and the third roll may rotate at a ratio of 1:1.5 to 1:5.

In the present invention, the device for preparing the molecular association is not limited to the above method, and it is natural that any device capable of applying a shear stress to an organic material, an inorganic material or a salt thereof may be freely deformed.

### Molecular association of an organic material, an inorganic material or a salt thereof

The present inventors have confirmed a phenomenon in which when drug molecules as pharmacologically active ingredients were dissolved in a solvent and then the molecules were brought very close to each other, polar groups in the molecules interacted to form a molecular association and behaved as if they were a single entity.

From the above, the present inventors have confirmed that when the polar groups interact with each other due to molecular proximity, the drug molecules are hydrophobic as a whole, but the dispersity may be increased because the surface tension decreases as the size of the molecular association in which these drug molecules are bonded decreases.

The present inventors have confirmed that a molecular association in which molecules with new properties are bonded may be prepared through various methods of reducing an intermolecular distance, such as the preparation of a molecular association using pores in powder or the approach of molecules using flexible rolls, and have completed the present invention.

Accordingly, the molecular association prepared by the apparatus as mentioned in this specification has the following characteristics:
First, the nano-molecular association according to the present invention is characterized in that the dispersity of the molecular association and the solubility of an organic material, an inorganic material or a salt thereof, which is a precursor of the molecular aggregate, are different from each other, by applying a shear stress to a solution containing the organic material, inorganic material or salt thereof, which is a precursor of the molecular aggregate, to reduce an intermolecular distance of the organic material, inorganic material or salt thereof very close, thereby preparing the molecular aggregate.

It is a well-known fact that the solubility of a material in water and solvent occurs through a process called solvation, and is known to always have the same value when the temperature and pressure are the same, so it is commonly called a thermodynamic property.

It can be seen that the molecular association provided in the present invention is a material that is physicochemically different from an organic material, an inorganic material or a salt thereof itself (i.e., precursor of the molecular aggregate) in terms of polarity of the surface, size and the like, and it can be predicted that changes caused by the formation of this molecular association will cause changes in physical properties such as solubility, known as thermodynamic properties.

Accordingly, the molecular association of the present invention is characterized in that when comparing the aqueous solubility A of an organic material, an inorganic material or a salt thereof, which is a precursor of the molecular aggregate, with the aqueous dispersity B of the molecular aggregate, the value of dispersity B/solubility A is more than 1. In this case, the value of dispersity B/solubility A of 1 or more means that the dispersity of the molecular association of the present invention is increased compared to the solubility of the organic material, inorganic material or salt thereof, which is a precursor of the molecular association of the present invention.

In the present invention, dispersion in the dispersity means that an association of several or more molecules does not precipitate by gravity and the number of molecules does not continuously increase due to surface interaction with the dispersion medium, and the dispersity is a measure of the degree of this dispersion. In addition, the solubility refers to the maximum amount of solute that maintains a reduced state to a size of a molecular state in a specific solution. The dispersity or solubility is determined based on when a material in powder form is used in the same volume of solution at the same temperature.

In the present invention, the method of measuring the solubility and dispersity is not particularly limited as long as it is a measurement method used in the art, but it may be preferably measured using an HPLC method. This means a method of measuring the solubility and dispersity by adding an excessive amount of solute to a solvent to produce a supersaturated solution, and then leaving it for a sufficient time at a predetermined temperature, and separating the liquid phase therefrom through a filter, and then measuring the mass of a pharmacologically active ingredient such as an organic material, an inorganic material or a salt thereof dissolved in the liquid phase, or the molecular association of the present invention. As a specific example, a supersaturated solution is produced, and then sufficiently left at 25°C, and the liquid phase is separated therefrom through a filter, and then the mass of a pharmacologically active ingredient such as an organic material, an inorganic material or a salt thereof dissolved in the liquid phase, or the molecular association of the present invention may be measured.

In the present invention, the value of dispersity B/solubility A may vary depending on the type of a pharmacologically active ingredient such as an organic material, an inorganic material or a salt thereof, and may be, for example, in excess of 1.0, 1.2 or more, 1.4 or more, 1.5 or more, 1.7 or more, 1.8 or more, 1.85 or more, 2.0 or more, 2.5 or more, 3.0 or more, 4.0 or more, 5.0 or more, 6.0 or more, 7.0 or more, 8.0 or more, 9.0 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 40 or more, 50 or more, 60 or more, 70 or more, 80 or more, 80 or more, or 100 or more, and its upper limit is not particularly limited, but may be 1000 or less.

Specifically, when the pharmacologically active ingredient is adenosine, the value of dispersity B/solubility A may be 1.5 or more, 1.7 or more, or 1.8 or more.

In addition, when the pharmacologically active ingredient is cyclosporin A, the value of dispersity B/solubility A may be 2.0 or more, 2.5 or more, or 3.0 or more.

In addition, when the pharmacologically active ingredient is niclosamide, the value of dispersity B/solubility A may be 15 or more, 18 or more, or 20 or more.

In addition, when the pharmacologically active ingredient is DCF-DA, the value of dispersity B/solubility A may be 25 or more, 28 or more, or 31 or more.

In addition, when the pharmacologically active ingredient is efinaconazole, the value of dispersity B/solubility A may be 2.0 or more, 2.5 or more, or 3.0 or more.

In addition, when the pharmacologically active ingredient is tacrolimus, the value of dispersity B/solubility A may be in excess of 1.0, 1.2 or more, 1.4 or more, or 1.5 or more.

In addition, the nano-molecular association according to the present invention has the following characteristics.

First, the nano-molecular association according to the present invention is characterized in that it is amorphous while having a nanoparticle size, by applying a shear stress to a solution containing an organic material, an inorganic material or a salt thereof, which is a precursor of the molecular aggregate, to reduce an intermolecular distance of the organic material, inorganic material or salt thereof very close, thereby preparing the molecular aggregate. That is, a shear stress is applied between molecules with the same structure to form a nano-sized amorphous molecular association in which they are physically bonded to each other.

In addition, the measured values by chromatography of the molecular association and the organic material, inorganic material or salt thereof, which is a precursor of the molecular aggregate, are the same, and the measured values by spectroscopy of the molecular association and the pharmacologically active ingredient, which is a precursor of the molecular aggregate, have different characteristics.

Common methods for determining the structure of a material include methods through various instrumental analyses, such as elemental analysis, FT-IR, NMR, UV spectroscopy, X-ray, and differential scanning calorimeter (DSC). Among them, NMR and FT-IR are the analytical methods that best show the chemical structure, that is, the atoms constituting the molecule and their connections. Usually, X-ray or DSC is a method widely used to investigate secondary structures such as crystal structures formed by molecules, such as crystal structures. In addition, the microstructure is also analyzed using a scanning electron microscope or a transmission electron microscope.

Among the methods mentioned, NMR may inform the electronic environment of an atomic nucleus, which is determined by the electronic structure of a molecule. The result of the present invention relates to a method for preparing a certain compound and its physical structure, and NMR provides very useful information as a method for determine whether there is no chemical change in the process of forming a physical structure. In the absence of formation or disappearance of chemical bonds, a compound and its physical structure basically have similar NMR spectra. Of course, in order to determine how close the compounds are to each other to form a molecular aggregate, an intermolecular distance of compounds usually located at a distance of 0.5 nm was roughly measured through the nuclear Overhauser effect (NOE) or the two-dimensional NOESY spectrum.

In addition, according to an example of the nano-molecular association according to the present invention, an intermolecular distance of the nano-molecular association may be 10 Å or less. The intermolecular distance refers to a value obtained by measuring the average distance between these molecules based on the molecules constituting a molecular aggregate, and may be measured using, for example, NOE of NMR. When a single hydrogen is excited using a pulse, the phenomenon in which the intensity of hydrogen at a close distance increases is called nuclear Overhauser effect (NOE), which is inversely proportional to the six squares of the distance between hydrogen nuclei. Thus, if intramolecular hydrogens are at a distance, they do not contribute to the increase in peak intensity due to NOE. However, when the molecules come very close together and the distance between intermolecular hydrogens is closer than the distance between intramolecular hydrogens, NOE is observed, indicating that an intermolecular distance becomes very close. It is generally known that NOE is observed when the distance is closer than 1 nm.

In the present invention, it has also been confirmed that the sodium deoxycholate (NaDC) compound and the molecular association obtained in the present invention are gathered on the scale of angstrom (10⁻¹⁰ m) due to the very close intermolecular distance through two-dimensional NOESY NMR.

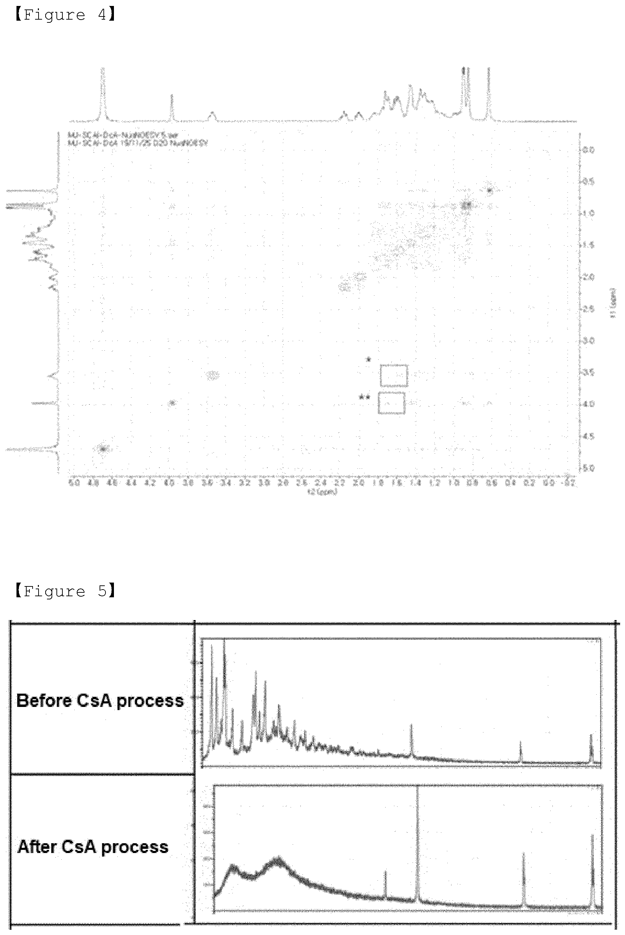

In Figures 3 and 4, the NOESY, which is a two-dimensional NOE spectrum, for NaDC molecules and a molecular association thereof, are shown, respectively. In Figures 3 and 4, parts marked with * and ** indicate the same positions. It can be seen that off-diagonal peaks do not appear in the regions marked with * and ** in the NaDC molecule itself in Figure 3, whereas off-diagonal peaks appear in the regions marked with * and ** in the molecular association of NaDC in Figure 4. This indicates that the distance between two nuclei was far from each other in pure NaDC, but the distance between the two nuclei became closer as the molecular association was formed. From the above, it can be seen that when the molecular association is formed, the intermolecular distance becomes very close. Since it is generally known that the distance required for two hydrogens to exhibit NOE is physically about 6 Å (Angstrom), it can be seen that an intermolecular distance to form the molecular association of the present invention is also within the range of about 6 Å (Angstrom).

In summary, the reason why peaks that did not appear due to the long distance in the molecule itself appear in the nano-molecular association provided by the present invention is caused by the change in the physical position of molecules of an organic material, an inorganic material or a salt thereof bonded to the nano-molecular aggregate. That is, this indicates that an intermolecular distance in the molecular association is very close.

The peak position in the NMR spectrum refers to a frequency at which rotational motion is performed depending on the intensity of a magnetic field applied to an atomic nucleus in a molecule consisting of atomic bonds as described above. The intensity of the magnetic field applied to the atomic nucleus varies depending on the property of the surrounding functional groups to push and pull electrons, and as a result, when the intensity of the magnetic field applied to the nucleus increases, it shifts to a higher frequency, and conversely, when the intensity of the magnetic field felt by the nucleus becomes weaker due to the large number of electrons around the nucleus, it reduces in the direction where the frequency of rotational motion of the nucleus is lower. When an association of molecules is physically formed, the density of the electron cloud of the atomic nucleus varies depending on the distance between the molecules with strong interactions. In another method, in a nucleus near a phenyl ring (C₆H₅-) where a ring current is formed under a magnetic field, the intensity of the magnetic field is locally changed by the ring current, and thus the number of revolutions of the nucleus is changed. In addition, even when the electron density is high due to the double bond, such as C=O, the position of the peak may shift up field or down field because the intensity of the magnetic field varies depending on the relative position of the surrounding nucleus and C=O.

In the case of the molecular association prepared according to the present invention, it is physically bonded by the interaction between molecules even without separate binders or additives, and may substantially consist of an organic material or an inorganic material.

Judging from these points, when comparing the result values measured by NMR of the molecular association prepared according to the present invention and the organic material or inorganic material, which is a precursor of the nano-molecular aggregate, it can be seen that the peak position of the NMR spectrum is partially changed, and this is because there is no change in chemical structure between the nano-molecular association and the organic material or inorganic material, which is a precursor of the nano-molecular aggregate, but a change in physical structure occurs. Specifically, when comparing the result values measured by NMR of the nano-molecular association and the organic material or inorganic material, which is a precursor of the nano-molecular aggregate, it may be determined that they are different when the peak shift is 0.005 ppm or more based on 1H NMR.

On the other hand, in the case of FT-IR, it is known that since the density of electrons constituting the bonds in the molecule is localized, a change in peak appears well when a change in dipole moment occurs during molecular motion, and it is known that peaks in the spectrum are excited by various molecular motions such as stretching and bending. When the polarity of the molecular bond is formed by heteroatom, a lot of peak changes appear. For example, an ether bond (C-O), which is a primary bond between carbon and oxygen, or a carbonyl bond (C=O), which is a secondary bond, shows a stretching band by an infrared laser. In addition, even in the case of carbon and hydrogen, a stretching band appears because it is a bond between different molecules. In the case of stretching, it may be explained by a model in which two masses are connected by a spring, and when the energy to excite the movement of the spring is supplied by infrared rays, peaks appear in the spectrum.

Even when several carbon atoms are connected to each other, various bending peaks such as in-plane vibration and out-of-plane vibration appear when six atoms are bonded, such as in benzene. In particular, in the case of bending motion, various types of molecular motions may be observed by FT-IR spectrum. For example, rocking that moves left and right, scissoring that moves like scissors, wagging that swings back and forth on a plane, twisting/torsion that repeats twisting, and the like occur.

Structures of strongly polar molecules may undergo interactions such as hydrogen bonding, polar interactions or pi-pi stacking, and these interactions cause changes in the position and intensity of peaks in the FT-IR spectrum. Thus, even in the same molecular bonding, the position and intensity of peaks may change depending on the surrounding environment. That is, it may be seen that they are the same molecule when the FT-IR spectrum is the same. In particular, 400 cm⁻¹ to 700 cm⁻¹ is called a fingerprint zone, and if peaks are the same in this zone, they are regarded as the same compound.

It is an object of the present invention to form compounds into a structure of a molecular aggregate, which is a physical aggregate. When the compounds are formed into a molecular aggregate, which is a physical aggregate, in this way, an intermolecular distance in the association is very close, within about 10 Å or 5 Å. It has been found that compound bonds in molecular aggregates undergo molecular motions such as stretching or bending when intramolecular bonds are excited by infrared rays, and in this case, due to another compound that exists within a very close distance from a compound, cases involving various changes, such as the appearance or disappearance of peaks that did not appear in the original compound spectrum, a change in the position of a peak or a decrease or increase in intensity of a peak, appear in the molecular association of the present invention.

That is, when one or more peaks appear or disappear based on the result values measured by the FT-IR, it may be determined that they are different.

When comparing the result values measured by FT-IR of the nano-molecular association prepared according to the present invention and the organic material or inorganic material, which is a precursor of the nano-molecular aggregate, it can be seen that one or more peaks of the FT-IR spectrum appear or disappear, and this is because there is no change in chemical structure between the nano-molecular association and the organic material or inorganic material, which is a precursor of the nano-molecular aggregate, but a change in physical structure occurs. In addition, when comparing the result values measured by FT-IR of the nano-molecular association and the organic material or inorganic material, which is a precursor of the nano-molecular aggregate, it may be determined that they are different when one or more peak positions differ by 5 cm⁻¹ or more. That is, the conversion of compounds into a molecular association is due to a physical action, and this does not mean that a new chemical bond is formed or a chemical bond disappears. Therefore, this change of the spectrum may well explain the fact that a molecular association with a new structure, which was not previously available, was created in the present invention.

However, the chemical structure of the nano-molecular association prepared according to the present invention and the organic material or inorganic material, which is a precursor of the nano-molecular aggregate, is the same as described above. This can be seen from the fact that chromatographic analysis was performed on the nano-molecular association and the organic material or inorganic material, which is a precursor of the nano-molecular aggregate, and the measured values were the same. The measured values by chromatography may be the result values measured by high-performance liquid chromatography (HPLC), and it is observed that the organic material or inorganic material and the molecular association thereof, which is the result provided by the present invention, show peaks at almost the same position and have a retention time within about 10 % (i.e., ±5%). In general, when the column and the mobile phase and the stationary phase are the same, peaks appearing at a time within 10% may be determined as the same material.

Like this, since the nano-molecular association and the organic material or inorganic material, which is a precursor of the nano-molecular aggregate, have different physical structures, most of the peaks of the two target materials are similar or some may be changed in the NMR spectrum, and various types of changes such as appearance and disappearance of peaks and changes in peak intensity may appear in FT-IR. In addition, since the nano-molecular association and the organic material or inorganic material, which is a precursor of the nano-molecular aggregate, have the same chemical structure, the measured values by chromatography may be the same.

In the present invention, NMR measurement of the nano-molecular association and the organic material or inorganic material, which is a precursor of the nano-molecular aggregate, is not particularly limited, but may be performed using, for example, a Bruker 400 MHz Avance.

In the present invention, FT-IR measurement of the nano-molecular association and the organic material or inorganic material, which is a precursor of the nano-molecular aggregate, is not particularly limited, but may be performed using, for example, a Bruker Alpha 2 ATR.

Next, another molecular association derived from drug molecules according to the present invention is characterized in that a shear stress is applied to a solution containing a salt of organic material or inorganic material as a precursor of the nano-molecular association to prepare a nano-molecular association with a structure in which the salt of organic material or inorganic material is physically bonded.

The molecular association prepared in this way is a molecular association in which a pharmacologically active ingredient and a water-soluble compound are bonded, and may be characterized in that the measured values by chromatography of a salt of organic material or inorganic material, which is a precursor of the nano-molecular aggregate, and a molecular association in which the salt of organic material or inorganic material is bonded are the same, and the measured values by spectroscopy of the salt of organic material or inorganic material, which is a precursor of the nano-molecular aggregate, and the molecular association in which the salt of organic material or inorganic material is bonded are different.

In the nano-molecular aggregate, the measured value by chromatography and the measured value by spectroscopy are the same as mentioned above.

Likewise, it is physically bonded by the interaction between molecules even without separate binders or additives, and may substantially consist of a salt of organic material or inorganic material.

In the present invention, the salt of organic material or inorganic material may be used as a pharmacologically active ingredient, and the salt of the above-mentioned organic material or inorganic material may be used.

As described above, since the salt of organic material or inorganic material, which is a precursor of the nano-molecular aggregate, and the molecular association in which the salt of organic material or inorganic material is physically bonded have different physical structures, most of the peaks of the two target materials are similar and some may be changed in the NMR spectrum, and various types of changes such as appearance and disappearance of peaks and changes in peak intensity may appear in FT-IR.

For example, when comparing the result values measured by NMR of the salt of organic material or inorganic material, which is a precursor of the nano-molecular aggregate, and the molecular association in which the salt of organic material or inorganic material is bonded, it may be determined that they are different when the peak position changes, and specifically, when comparing the result values measured by NMR of the salt of organic material or inorganic material, which is a precursor of the nano-molecular aggregate, and the molecular association in which the salt of organic material or inorganic material is bonded, it may be determined that they are different when the peak shift is 0.005 ppm or more based on 1H NMR.

In addition, when comparing the result values measured by FT-IR of the salt of organic material or inorganic material, which is a precursor of the nano-molecular aggregate, and the molecular association in which the salt of organic material or inorganic material is bonded, they may be determined to be different when one or more peaks appear or disappear, and specifically, when comparing the result values measured by FT-IR of the salt of organic material or inorganic material, which is a precursor of the nano-molecular aggregate, and the molecular association in which the salt of organic material or inorganic material is bonded, they may be determined to be different when one or more peaks differ by 5 cm⁻¹ or more.

In addition, since the salt of organic material or inorganic material, which is a precursor of the nano-molecular aggregate, and the molecular association in which the salt of organic material or inorganic material is bonded have the same chemical structure, the measured values by chromatography may be the same. Specifically, they may be determined to be the same, when the result values measured by HPLC of the salt of organic material or inorganic material, which is a precursor of the nano-molecular aggregate, and the molecular association in which the salt of organic material or inorganic material is bonded have a retention time within 10%.

In addition, since the particle size of the nano-molecular association according to the present invention is very small, the average particle diameter may be 50 nm or less, preferably may be 30 nm or less, more preferably may be 20 nm or less, very preferably may be 15 nm or less, and most preferably may be 10 nm or less, or 5 nm or less. The average particle diameter may be measured through a diffraction experiment, and preferably may be measured using small angle neutron scattering (SANS). Also, images may be measured using transmission electron microscopy. When the average particle diameter of the nano-molecular association is more than 50 nm, there are problems that dispersibility is poor and transparency and permeability are poor. In addition, the lower limit of the average particle diameter of the nano-molecular association is not particularly limited, but may be about 1 nm or more.

Hereinafter, the preparation method of the present invention will be described in more detail through examples of the present invention. It will go without saying that the present invention is not limited to these examples.

### EXAMPLES

### Apparatus used

3 roll mill

| Model | | TX3051 Series | |
|---|---|---|---|
| Motor | | 120W (3P/220V) | |
| RPM | | Max. 360 | |
| Dimension (mm) | | 310(V) × 250(D) × 360(H) | |
| Roller | Rotation Rate | 9(P) : 3(M) : 1( R) | |
| | Temp. Range | Non (Room Temp) | |
| | Gap | 0 ~ 300 mm | |
| | Gap Control Method | Dial Rotation Manual Type (15µ m/1 Rotation Fine Control) | |
| | Display | Digital (µ m) LCD Display (P-M. M-R) | |
| Calibration | | Independent Calibration, Front/Back, Left/Right | |

### Preparation

### [Example 1]

1 g of adenosine (Aldrich Co. Ltd.) was dissolved in 99 mL of ethanol to prepare an adenosine solution with a concentration of about 0.2%. As shown in Figure 1, a roll mill composed of two rolls, roll A and roll B, was prepared, and then the prepared aqueous adenosine solution was put between rolls A and B at an input speed of 50 ml/min. The rotational speed of the roll A was adjusted to 100 rpm and the rotational speed of the roll B was adjusted to 300 rpm, and the distance between the rolls A and B was set to 10 um. The ethanol adenosine molecular association solution obtained between the rolls were mixed with distilled water, and then ethanol was removed using a vacuum dryer to obtain a transparent aqueous adenosine dispersion.

The obtained aqueous solution was frozen at -50°C, and then freeze-dried at the temperature of -70°C under a pressure of 0.1 bar for 48 hours to remove water, and as a result, a molecular association powder of adenosine was obtained.

### [Comparative Example 1]

An adenosine powder was obtained through the same process as in Example 1, except that an adenosine solution was prepared and the roll process for preparing a molecular association thereof was not performed.

### [Example 2]

A molecular association powder of cyclosporin A was obtained in the same manner as in Example 1, except that cyclosporine A (TEVA) was used instead of adenosine.

### [Comparative Example 2]

Cyclosporin A powder was obtained through the same process as in Example 2, except that cyclosporin A solution was prepared and the roll process for preparing a molecular association thereof was not performed.

### [Example 3]

A yellow molecular association powder of niclosamide was obtained in the same manner as in Example 1, except that niclosamide (Sigma-Aldrich Co. Ltd.) was used instead of adenosine.

### [Comparative Example 3]

A niclosamide powder was obtained through the same process as in Example 3, except that a niclosamide solution was prepared and the roll process for preparing a molecular association thereof was not performed.

### [Example 4]

19.8 mg of DCF-DA (2',7'-dichlorofluorescin diacetate; Sigma-Aldrich Co. Ltd.) was dissolved in 10 mL of water to prepare an aqueous DCF-DA solution with a concentration of about 0.2%. As shown in Figure 1, a roll mill composed of two rolls, roll A and roll B, was prepared, and then the prepared DCF-DA solution was put between rolls A and B at an input speed of 50 ml/min. The rotational speed of the roll A was adjusted to 100 rpm and the rotational speed of the roll B was adjusted to 300 rpm, and the distance between the rolls A and B was set to 10 um.

The obtained aqueous solution was frozen at -50°C, and then freeze-dried at the temperature of -70°C under a pressure of 0.1 bar for 48 hours to remove water, and as a result, a molecular association powder of DCF-DA was obtained.

### [Comparative Example 4]

DCF-DA powder was obtained through the same process as in Example 4, except that DCF-DA was prepared as an aqueous solution and the roll process for preparing a molecular association thereof was not performed.

### [Example 5]

A white molecular association powder of epinaconazole was obtained in the same manner as in Example 1, except that epinaconazole (Sigma-Aldrich Co. Ltd.) was used instead of adenosine in Example 1.

### [Comparative Example 5]

A white epinaconazole powder was obtained through the same process as in Example 5, except that an epinaconazole ethanol solution was prepared and the roll process for preparing a molecular association thereof was not performed.

### [Example 6]

A white molecular association powder of tacrolimus was obtained in the same manner as in Example 1, except that tacrolimus (Sigma-Aldrich Co. Ltd.) was used instead of adenosine.

### [Comparative Example 6]

A white tacrolimus powder was obtained through the same process as in Example 6, except that a tacrolimus solution was prepared and the roll process for preparing a molecular association thereof was not performed.

### Experimental Example 1: Comparison of XRD structures in Example 2 and Comparative Example 2

The XRD measurement results of the molecular association in Example 2 of the present invention prepared using cyclosporin A and the cyclosporin A itself in Comparative Example 2 were compared and are shown in Figure 5. As can be seen through the comparison of the graphs, except for the peaks appearing by the holder (SUS material) holding the specimen (peaks appearing identically on the right side of the graphs), it can be seen that the API (cyclosporine A) itself in Comparative Example 2 before the process exists as a crystal form (sharp peaks on the left side), whereas the molecular association in Example 2 of the present invention after the process changes to an amorphous form (amorphous peaks on the left) as the physical structure changes.

In the case of a crystalline structure rather than an amorphous structure such as the molecular association of the present application, peaks may change as the size of the internal lattice changes, but this is also just a change from the existing crystal form to another crystal form, and it does not change to an amorphous form as in the present invention.

Therefore, it can be seen from Figure 5 that the molecular association of the present invention does not change to a sorbate form, but the physical structure itself changes to an amorphous form.

### Experimental Example 2: Comparison of DSC measurement experiment of Example 2 and Comparative Example 2

In order to clearly prove that the molecular association of the present application was not crystallized (sorbated), the applicant of the present application conducted the following DSC measurement experiment.

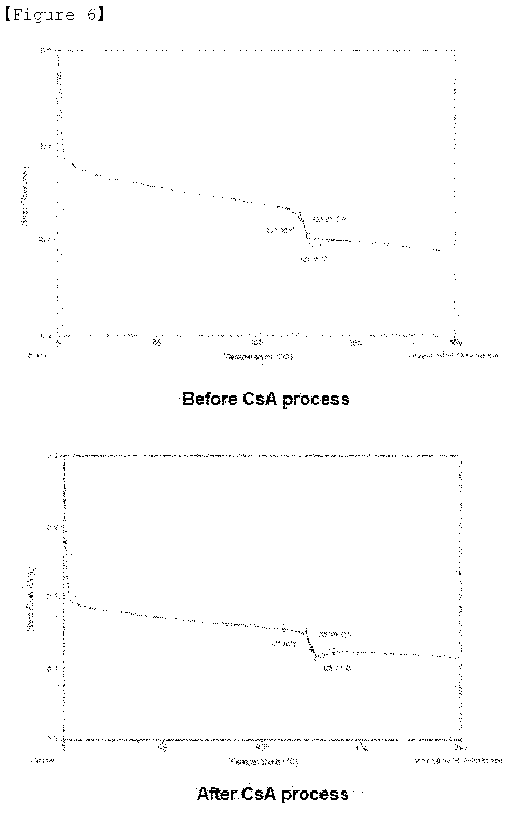

Specifically, the DSC measurement results of the molecular association in Example 2 of the present invention prepared using cyclosporin A as API and the cyclosporin A itself in Comparative Example 2 were compared and are shown in Figure 6. If the molecular association that has undergone the process of the present invention is sorbate, a peak in which the solvent (the solvent used in the present invention is water or ethanol) blows away near the boiling point (100°C for water, about 60°C for ethanol) should appear on the DSC graph, but such a peak does not appear at all in the DSC measurement result data shown in Figure 6. Therefore, it can be clearly seen that the difference between the measured values by spectroscopy of the molecular association of the present application and the pharmacologically active ingredient, which is a precursor of the nano-molecular aggregate, is due to a change in physical structure, not a change caused by sorbation.

### Experimental Example 3: Confirmation of particle pictures of Examples

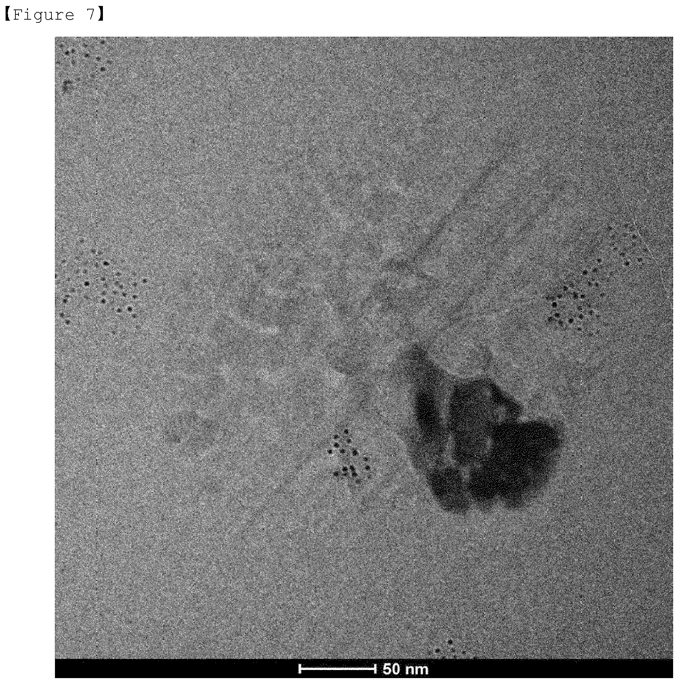

In order to confirm that the molecular association of the present application is a molecular association structure of API, the applicant of the present application took TEM pictures of the molecular association in Example 2 prepared using cyclosporin A as API. First, a TEM picture of the molecular association in Example 2 prepared using cyclosporin A as API was taken and is shown in Figure 7. Cyclosporin A itself cannot be confirmed by TEM because it is not well soluble in water, but as can be seen in the TEM picture of Figure 7, it can be confirmed that the molecular association according to the present invention is a structure in which several APIs are physically bonded.

Therefore, it can be clearly seen that the molecular association according to the present invention is a molecular association structure in which a plurality of APIs are physically bonded.

### Experimental Example 4: Comparison of solubility performance of Examples and Comparative Examples

The dispersity of the molecular association (B) prepared in Examples 1 to 6 above and the solubility of the pharmacologically active material itself (A) prepared in Comparative Examples 1 to 6 were compared. Each sample was stirred at 25°C for 10 hours and filtered through a 0.2 micron filter, and then the solubility was determined through peak integration using the concentration obtained through HPLC (e2695 from Waters) for the dissolved amount of the APIs of Comparative Examples or the dispersed amount of the molecular aggregates of Examples.

**[Table 1]**

| Classification | Material | Aqueous solubility (dispersity) % (wt/v) |
|---|---|---|
| 1 | Adenosine (A) | 0.27 |
| | Molecular association of adenosine (B) | 0.5 |
| | B/A | 1.85 |
| 2 | Cyclosporin A (A) | 0.0007 |
| | Molecular association of cyclosporin A (B) | 0.0022 |
| | B/A | 3.14 |
| 3 | Niclosamide (A) | 0.0033 |
| | Molecular association of niclosamide (B) | 0.07 |
| | (B/A) | 21.21 |
| 4 | DCF-DA (A) | 0.004 |
| | Molecular association of DCF-DA (B) | 0.13 |
| | (B/A) | 32.5 |
| | Epinaconazole (A) | 0.321 |
| 5 | Molecular association of Epinaconazole (B) | 1.0 |
| | (B/A) | 3.1 |
| 6 | Tacrolimus (A) | 7.6 |
| | Molecular association of tacrolimus (B) | 11.2 |
| | (B/A) | 1.5 |

As described above, it can be seen that the substructure of the pharmacologically active material is greatly improved in terms of the dispersibility in the solvent and water compared to the pharmacologically active material itself, which will not only become a very important feature when formulating medicines in the future, but also become a great advantage in enhancing its efficacy.

### Experimental Example 5: Comparison of cell permeation performance of Example 4 and Comparative Example 4

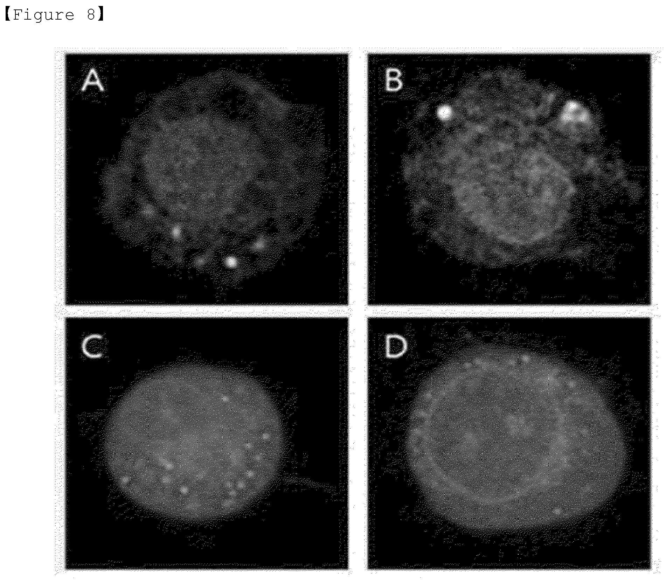

The cell permeation performance of the DCF-DA molecular association in powder form prepared in Example 4 and the DCF-DA itself in Comparative Example 4 was compared. Suspension cells (MV-4-11 human macrophages) were grown in dishes to fill up to 50%. DCF-DA in Comparative Example 4 was dissolved in ethanol, and the DCF-DA molecular association in Example 4 was dissolved in distilled water, and the cells were treated with each of them at the same concentration of 0.005% for 30 minutes, and then the cells were washed with phosphate buffer solution (PBS) to remove extracellular DCF-DA and to stop the entry by diffusion. The cells were stabilized by culture for an additional 30 minutes, and then treated with 0.03% hydrogen peroxide (H₂O₂), and intracellular fluorescent DCFs were photographed by 3D hologram and fluorescence using a 3D microscope (HT-2H from Tomocube, Inc.), and are shown in Figure 8. As a result of the experiment, it could be confirmed that the DCF-DA molecular aggregate-treated group in Example 4 as shown in C and D of Figure 8 showed much stronger fluorescence compared to the DCF-DA-treated group in Comparative Example 4 as shown in A and B of Figure 8. Therefore, it could be confirmed that the permeability of the molecular association prepared according to the present invention into the phospholipid membrane was much higher.

## Claims

1. A nano-molecular association in which an organic material or an inorganic material is physically bonded, **characterized in that**:
when formed as a composition comprising water in the nano-molecular aggregate, the nano-molecular association in the composition has an aggregated structure;
the average particle diameter of the nano-molecular association is 50 nm or less; and
when comparing the aqueous solubility (A) of a pharmacologically active ingredient with the aqueous dispersity (B) of the nano-molecular aggregate, the value of dispersity (B)/solubility (A) is 1.2 or more.

2. The nano-molecular association according to claim 1, **characterized in that** the nano-molecular association is amorphous.

3. The nano-molecular association according to claim 1, **characterized in that** the organic material or inorganic material is any one or more selected from the group consisting of adenosine, cyclosporin A, niclosamide, paclitaxel, docetaxel, decursin, meloxicam, itraconazole, celecoxib, capecitabine, travo, prost, isoflavone, diclofenac sodium, sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, deoxycholic acid, ursodeoxycholic acid, sodium or potassium salt of deoxycholic acid, sodium or potassium salt of ursodeoxycholic acid, predimesolone, eucalyptus oil, lavender oil, lemon oil, sandalwood oil, rosemary oil, chamomile oil, cinnamon oil, orange oil, alpha-bisabolol, vitamin A (retinol), vitamin E, tocopheryl acetate, DCF-DA, efinaconazole, vitamin D, vitamin F, and derivatives thereof.

4. The nano-molecular association according to claim 1, **characterized in that** the nano-molecular association has an intermolecular distance of 10 Å or less.

5. The nano-molecular association according to claim 1, **characterized in that** when the organic material or inorganic material is adenosine, the value of dispersity (B)/solubility (A) is 1.5 or more.

6. The nano-molecular association according to claim 1, **characterized in that** when the organic material or inorganic material is cyclosporin A, the value of dispersity (B)/solubility (A) is 2.0 or more.

7. The nano-molecular association according to claim 1, **characterized in that** when the organic material or inorganic material is niclosamide, the value of dispersity (B)/solubility (A) is 15 or more.

8. The nano-molecular association according to claim 1, **characterized in that** when the organic material or inorganic material is DCF-DA, the value of dispersity (B)/solubility (A) is 25 or more.

9. The nano-molecular association according to claim 1, **characterized in that** when the organic material or inorganic material is efinaconazole, the value of dispersity (B)/solubility (A) is 2.0 or more.

10. The nano-molecular association according to claim 1, **characterized in that** when the organic material or inorganic material is tacrolimus, the value of dispersity (B)/solubility (A) is 1.2 or more.

11. The nano-molecular association according to claim 1, **characterized in that** the nano-molecular association consists of the organic material or inorganic material.
